# EUROPEAN PATENT APPLICATION

(11) **EP 4 506 466 A1**
(43) Date of publication of application: **12.02.2025**
(21) Application number: 22934357.9
(22) Date of filing: 02.04.2022
(51) Int. Cl.: C12Q 1/6851, C12N 15/11

(54) **APPLICATION OF MARKER GENES IN DETECTING MULTIPOTENT STEM CELL RESIDUES, DETECTION METHOD AND KIT**

(71) Applicant: Iregene Therapeutics Ltd, Wuhan, Hubei 430000 (CN)
(72) Inventor: CAI, Meng, Wuhan, Hubei 430000 (CN); WEI, Jun, Wuhan, Hubei 430000 (CN); MAO, WeiBing, Wuhan, Hubei 430000 (CN); ZHU, YaSha, Wuhan, Hubei 430000 (CN)
(74) Representative: Zaboliene, Reda
(86) International application number: PCT/CN2022/085015
(87) International publication number: WO 2023/184528

(57) **Abstract**

Disclosed are an application of a marker gene in detecting multipotent stem cell residues, a detection method and a kit. The marker gene comprises at least one of TDGF1 and TRIML2, and is a gene with high-specificity expression in human pluripotent stem cells. The screening of the marker gene is based on a high-throughput RNA sequencing technology. Genes with significantly up-regulated specific expression in pluripotent stem cells are screened from the pluripotent stem cells and derivative cell samples of the pluripotent stem cells, a group of candidate genes which can be used for identifying pluripotent stem cell residues are identified therefrom, and the marker gene is selected from the candidate genes for validation. The marker gene provided by the present invention can be used for detecting and identifying pluripotent stem cell residues in a derivative cell product of pluripotent stem cells for clinical treatment, so as to perform quality control on clinical cell products.

## Description

### Background of the Invention

### Field of the Invention

The present invention relates to the field of detecting residual pluripotent stem cells. Specifically, it involves the use of marker genes in detecting residual pluripotent stem cells, as well as the detection methods and reagent kits.

### Description of Related Art

Regenerative medicine refers to an emerging science that utilizes various new technologies to reconstruct aging or functionally impaired tissues and organs and to treat related diseases through multiple medical approaches. Major research directions in regenerative medicine include understanding the mechanisms of normal tissue characteristics and functions, the biological basis of post-injury repair, and the mechanisms of tissue and organ regeneration as well as the differentiation mechanisms of various stem cells, with the ultimate goal of developing effective biological therapies. The research methods in regenerative medicine integrate principles and methods from multiple disciplines, including life sciences, materials science, and clinical medicine, to provide clinical solutions for replacing, repairing, reconstructing, or regenerating various tissues and organs in the human body.

During the development of regenerative medicine, several core issues need to be addressed. Ideal regenerative medicine products, especially cell therapy products, should have the following characteristics: first, the cell source must be a single, indefinitely proliferating cell type; second, the cells must be amenable to further modification, such as correcting pathogenic genes using gene-editing tools; third, the final product must be uniform in nature. One of the early research topics in regenerative medicine is how to obtain initial cell sources suitable for cell therapy. Among these, embryonic stem cells (ESCs) are the most prominent cell type in early regenerative medicine research. ESCs are cells isolated from early embryos (before the blastocyst stage) or primordial germ cells. They have the ability to proliferate indefinitely in vitro, self-renew, and differentiate into multiple cell types. ESCs can be induced to differentiate into nearly all cell types in both in vitro and in vivo environments. However, obtaining and using these cells is highly controversial from an ethical standpoint, as embryonic stem cell research involves the destruction of embryos, which are considered a form of life before human formation in the womb. This ethical controversy significantly hinders the advancement and application of regenerative medicine.

In 2006, Shinya Yamanaka's team invented a "cocktail" method using four transcription factors-OCT4, SOX2, KLF4, and c-Myc-to successfully reprogram terminally differentiated skin fibroblasts into pluripotent stem cells, known as induced pluripotent stem cells (iPSCs) (Takahashi K, et al., Cell, 2006, 126(4): 663-676; Takahashi K and Yamanaka S, Cell, 2007, 131(5): 861-872). These cells possess differentiation potential similar to that of ESCs, capable of forming the three basic germ layers of human development-ectoderm, mesoderm, and endoderm-and ultimately differentiating into various adult cell types. This invention overcomes the ethical limitations of using human embryonic stem cells in medicine and addresses immune rejection issues in cell transplantation, greatly expanding the clinical potential of stem cell technology. Using totipotent or pluripotent stem cells, including ESCs and iPSCs, as starting materials for inducing ectodermal cell differentiation presents a new approach for clinical treatment and greatly expands the clinical application potential of ectodermal cells. In regenerative medicine alone, iPSCs are being investigated in clinical applications for neurodegenerative diseases, spinal cord injuries, Type 1 diabetes, and tumors.

However, pluripotent stem cells, including ESCs and iPSCs, have the ability to proliferate indefinitely and can form teratomas in vivo. Therefore, residual pluripotent stem cells must be removed from clinical products to ensure safety. Current methods for monitoring residual pluripotent stem cells have several limitations. For example, the cell culture method involves observing clones formed by residual cells after culturing for two weeks. This method is time-consuming and cannot eliminate false negatives due to spontaneous differentiation of pluripotent stem cells. Additionally, detecting residual pluripotent stem cells by identifying specific markers using flow cytometry has low resolution, with a detection accuracy of only 1%. A more common method is quantitative PCR or digital PCR to detect the expression of specific genes in pluripotent stem cells, thus determining the residual proportion. However, some currently used marker genes, such as Nanog and Lin28, while highly expressed in pluripotent stem cells, also show high expression in some derived cells, making them inaccurate markers for identifying residual pluripotent stem cells, with low detection accuracy (around 0.1%).

TDGF1, located on chromosome 3p21.3, has a gene length of 2 kb or 1.7 kb, containing 6 exons and 5 introns, or 4 exons and 5 introns, with an open reading frame of 564 bp. TDGF1 plays a crucial role during embryonic development, especially in stem cell renewal, multifunctional differentiation, and survival. During early embryonic development, the EGF-CFC protein acts as an essential co-receptor for transforming TGF-β family signals. TDGF1 and TGF-β family ligand Nodal are key regulatory factors in embryonic development and are markers of undifferentiated human embryonic stem cells, with minimal expression in normal adult tissues.

The TRIML2 gene encodes Tripartite Motif Family Like 2 protein. This protein may be regulated by the tumor suppressor p53 and potentially modulate p53 through enhancing p53-like ubiquitination modifications. Alternative splicing of this gene results in several transcript variants. TRIML2 shows significant expression variation in pluripotent stem cells and their derived neural stem cells (NSCs) and dopaminergic progenitor cells (DPCs), with high sensitivity, making it suitable for identifying residual pluripotent stem cells.

In existing technologies, markers such as Oct4, Nanog, and Lin28 (Sekine K., et al., Sci Rep, 2020, 10, 10293) are used to identify residual pluripotent stem cells. However, due to high expression levels of these genes in iPSC-derived cells such as NSCs and DPCs, with only minor differences compared to iPSCs, their sensitivity is insufficient, leading to higher clinical risks for pluripotent stem cell-derived products and unassured safety in clinical use.

### Summary of the Invention

The present invention aims to propose a method for the application of marker genes in detecting residual pluripotent stem cells, as well as detection methods and reagent kits, to address at least one of the numerous issues present in the prior art.

Specifically, the invention provides the following solution:
The invention proposes the use of marker genes in detecting residual pluripotent stem cells, characterized in that the marker genes are used to detect residual pluripotent stem cells in pluripotent stem cell-derived cells, with the marker genes including at least one of TDGF1 and TRIML2.

Further, the pluripotent stem cell-derived cells include neural stem cells, retinal ganglion cells, retinal pigment epithelial cells, liver cells, natural killer cells, and cardiomyocytes.

The invention provides a method for detecting residual pluripotent stem cells, characterized in that the method includes using at least one marker gene selected from TDGF1 and TRIML2 to detect the amount of residual pluripotent stem cells.

Further, the detection method involves quantitatively detecting the marker gene in the sample and comparing it with a standard of pluripotent stem cell-derived cells containing known quantities of pluripotent stem cells to obtain the amount of residual pluripotent stem cells.

Further, the detection method includes the following steps:
(1) Adding varying gradients of pluripotent stem cells to pluripotent stem cell-derived cells to create standards;
(2) Designing primers based on the sequences of the marker genes and performing quantitative PCR with the primers and the standards to create a standard curve for residual pluripotent stem cells in the derived cells;
(3) Performing quantitative PCR on the pluripotent stem cell-derived cells to detect the expression levels of the marker genes in the sample, and determining the residual pluripotent stem cell proportion based on the comparison of Ct values.

Preferably, the nucleotide sequences of the primers for the marker gene TDGF1 are SEQ ID NO: 3 and/or SEQ ID NO: 4.

Preferably, the nucleotide sequences of the primers for the marker gene TRIML2 are SEQ ID NO: 5 and/or SEQ ID NO: 6.

Preferably, in step (2), the qPCR detection program includes: pre-denaturation at 95°C for 120 seconds; PCR reaction with 40 cycles of 95°C for 10 seconds, 58°C for 20 seconds; followed by 94°C for 30 seconds.

A reagent kit for detecting residual pluripotent stem cells, characterized in that it includes primers designed based on the nucleotide sequences SEQ ID NO: 1 or SEQ ID NO: 2.

Preferably, the primers include at least one of the nucleotide sequences SEQ ID NO: 3-4 or SEQ ID NO: 5-6.

### Beneficial effects:

Compared to the prior art, the beneficial effects of the present invention are as follows:
1. The invention introduces new applications of TDGF1 and TRIML2 for detecting residual pluripotent stem cells in pluripotent stem cell-derived cells.
2. The invention utilizes transcriptome sequencing to identify genes that are highly expressed in pluripotent stem cells and lowly expressed in derived differentiated cells. These genes are then validated by quantitative PCR to confirm method reliability and determine detection sensitivity. Compared to markers such as Oct4, Nanog, and Lin28, the markers TDGF1 and TRIML2 selected by the invention have significantly lower background expression in iPSC-derived cells (e.g., NSC and DPC cells), resulting in much higher sensitivity, approximately 0.01% compared to 0.1% for the former markers. This greatly enhances the accuracy of residual pluripotent stem cell detection, reducing the clinical risk of pluripotent stem cell-derived products and significantly improving their safety for clinical use.
3. The invention provides a method applicable to a wide range of pluripotent stem cell-derived cells, including neural stem cells, retinal ganglion cells, retinal pigment epithelial cells, liver cells, natural killer cells, and cardiomyocytes, making it highly versatile for quality control in clinical cell products.

### Brief Description of the Figures

To clarify the technical solutions of the embodiments of the present invention, the following is a brief description of the drawings used in the embodiments. It should be understood that the following drawings illustrate only certain embodiments of the present invention and should not be considered as limiting the scope. Those skilled in the art can derive other relevant drawings based on these illustrations without inventive effort.

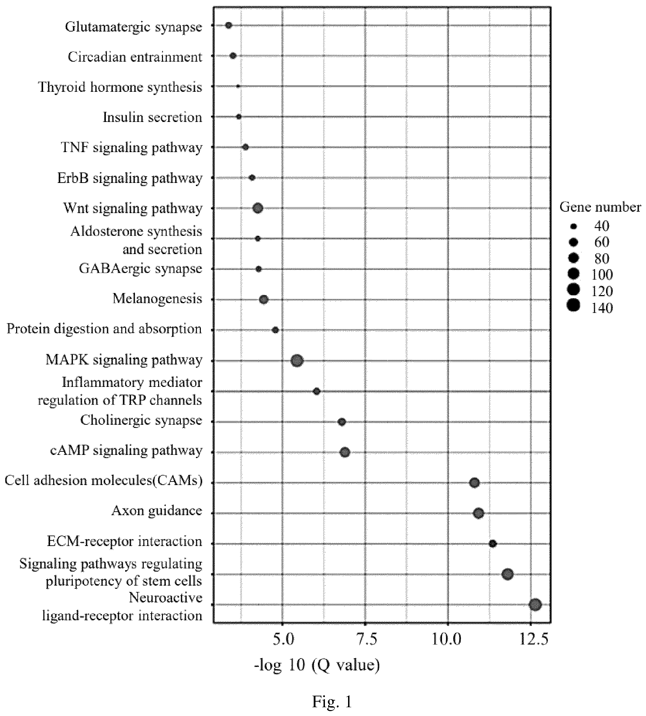

Figure 1 shows the results of the gene selection with high specificity expression in pluripotent stem cells through the GO pathway analysis database.

### Detailed Description of the Invention

The present invention proposes the use of marker genes in detecting residual pluripotent stem cells, where the marker genes are TDGF1 and/or TRIML2. The application of the present invention involves detecting residual pluripotent stem cells in pluripotent stem cell-derived cells, which include, but are not limited to, neural stem cells, retinal ganglion cells, retinal pigment epithelial cells, liver cells, natural killer cells, and cardiomyocytes.

In this specification, "detect/detecting/detection" refers to confirming the presence of residual pluripotent stem cells, but it also includes confirming their absence.

Embodiments of the present invention use quantitative fluorescence PCR to obtain Ct values of TDGF1 and TRIML2 gene expression in dopamine precursor cells (DPC) with residual pluripotent stem cell proportions of 0, 0.01%, 0.1%, and 1%. The invention also uses pluripotent stem cell-derived retinal progenitor cells (RPC), hepatic progenitor cells (HPC), and cardiomyocyte progenitor cells (CPC) as samples for validating expression levels. According to the method of the present invention, it provides a detection sensitivity of 0.01%, but the minimum residual detection amount is not limited to 0.01%.

The following examples are provided to illustrate the present invention but do not limit its scope. Modifications or substitutions made to the methods, steps, or conditions of the present invention without departing from its spirit and essence are within the scope of the invention.

### Experimental Materials

1. Samples for preparing standards: Human-derived dopamine precursor cells (DPC); pluripotent stem cells provided by our laboratory, cell batch number 20201225.
2. Samples to be tested: DPC-1, DPC-2, DPC-3 cells provided by the laboratory, cell batch numbers 20201007-1, 20201007-2, 20201007-3, respectively; retinal progenitor cells (RPC), hepatic progenitor cells (HPC), and cardiomyocyte progenitor cells (CPC) prepared and provided by our laboratory.

### Experimental Reagents

1. RNA extraction kit MiniBEST Universal RNA Extraction Kit, TAKARA, Code No. 9767.
2. Reverse transcription kit PrimeScript^{™} II 1st Strand cDNA Synthesis Kit, TAKARA, Code No. 6210A.
3. qPCR reagent TB Green^{®} Premix Ex Taq^{™} II (Tli RNaseH Plus), TAKARA, Code No. RR820A.

In the following examples, the method for extracting total mRNA is as follows:
1. Add 600 µL of Lysis Buffer RL (containing 50×DTT solution, final concentration 1%) to the collected cell pellet, and use a pipette to mix until no obvious sediment is seen in the lysate.
2. Incubate the lysate at room temperature for 2 minutes.
3. Add an equal volume of 70% ethanol to the lysate (precipitation may occur), and mix the solution thoroughly using a pipette.
4. Immediately transfer the entire mixture (including the precipitate) into the RNA nucleic acid purification column (Spin Column) with a 2 mL collection tube (if the volume of the mixture is greater than 600 µL, add in batches, with each batch not exceeding 600 µL).
5. Centrifuge at 12,000 rpm for 1 minute and discard the filtrate. Place the RNA nucleic acid purification column back into the 2 mL collection tube.
6. Add 500 µL of Buffer RWA to the RNA nucleic acid purification column, centrifuge at 12,000 rpm for 30 seconds, and discard the filtrate.
7. Add 600 µL of Buffer RWB to the RNA nucleic acid purification column, centrifuge at 12,000 rpm for 30 seconds, and discard the filtrate.

Note: Ensure that 100% ethanol has been added to Buffer RWB as specified.

Add Buffer RWB along the wall of the RNA nucleic acid purification column to help completely wash off the salts attached to the column wall.

8. DNase I digestion:
Using the gDNA Eraser nucleic acid purification column and RNA nucleic acid purification column in this kit can effectively remove most genomic DNA from cultured cells, with the extracted RNA containing minimal genomic DNA. If the RNA purity requirement is stringent, selective DNase I digestion can be performed on the RNA nucleic acid purification column membrane.
① Prepare DNase I reaction solution: Mix 5 µL of 10×DNase I Buffer, 4 µL of recombinant DNase I (RNase free, 5 U/µL), and 41 µL of RNase free dH₂O in a new 1.5 mL RNase Free tube.
② Add 50 µL of DNase I reaction solution to the center of the RNA nucleic acid purification column membrane, and incubate at room temperature for 15 minutes.
③ Add 350 µL of Buffer RWB to the center of the RNA nucleic acid purification column membrane, centrifuge at 12,000 rpm for 30 seconds, and discard the filtrate.

9. Repeat step 7.

10. Place the RNA nucleic acid purification column back into the 2 mL collection tube and centrifuge at 12,000 rpm for 2 minutes.

11. Place the RNA nucleic acid purification column onto a 1.5 mL RNase Free collection tube (provided in the kit), add 50 µL of RNase Free dH₂O or 0.1% Diethyl pyrocarbonate (DEPC) treated water to the center of the RNA nucleic acid purification column membrane, and incubate at room temperature for 5 minutes.

12. Centrifuge at 12,000 rpm for 2 minutes to elute RNA.

13. To increase RNA yield, add another 50 µL of RNase Free dH₂O or 0.1% DEPC treated water to the center of the RNA nucleic acid purification column membrane; for higher RNA concentration, the first eluate can be added back to the RNA nucleic acid purification column, incubate at room temperature for 5 minutes, and centrifuge at 12,000 rpm for 2 minutes to elute RNA.

In the following examples, the method for reverse transcribing total mRNA into cDNA is as follows:
1. Use a micro spectrophotometer to measure mRNA concentration and calculate the amount needed for reverse transcription, and prepare the reaction mixture according to the system shown in Table 1.

**Table 1**

| Reagent | Amount |
|---|---|
| Oligo dT Primer (50 µM) | 1 µL |
| dNTP Mix (10 mM) | 1 µL |
| Template RNA | 550 ng |
| RNase Free dH₂O | Up to 12.5µL |

2. After incubating at 65°C for 5 minutes, rapidly cool on ice.
(Note: This treatment denatures the template RNA and improves the efficiency of reverse transcription.)
3. Prepare the reverse transcription reaction mixture as shown in Table 2, with a total volume of 20 µL.

**Table 2**

| Reagent | Amount |
|---|---|
| Denatured reaction mixture (from Step 2) | 12.5 µL |
| RNase Inhibitor (40 U/µL) | 0.5 µL |
| 5× PrimeScript II Buffer | 4 µL |
| PrimeScript II RTase (200 U/µL) | 1 µL |

4. Mix slowly and thoroughly.
5. Perform the reverse transcription reaction under the following conditions: 60 minutes at 42°C, followed by 15 minutes at 70°C (to inactivate the enzyme), and then cool on ice.

The real-time fluorescence quantitative PCR detection method is as follows:
Prepare the reaction mixture for cDNA and qPCR reagents according to Table 3.

**Table 3**

| Reagent | Amount |
|---|---|
| Template | 3 µL |
| F/R Primers | 2 µL |
| 2× TB Green Premix Ex Taq II | 10 µL |
| RNase Free dH₂O | 5 µL |

Two-step PCR amplification program:
1. Initial denaturation at 95°C for 120 seconds.
2. PCR reaction, Number of Cycles: 40 cycles, with 10 seconds at 95°C and 20 seconds at 58°C; followed by melt curve stage.

### Example 1: Screening of Genes with Specific High Expression in Pluripotent Stem Cells

High-throughput transcriptome sequencing was performed on pluripotent stem cells and various types of cells derived from pluripotent stem cells (as described in Chinese Patent Publications CN108315301A and CN108103021A). The sequencing data were processed using software to remove sequence adapters and low-quality bases. The filtered sequences were then aligned to the human genome. The number of sequencing fragments corresponding to coding genes was calculated, and significant differences in gene expression between different cell types were tested. Based on the expression fold changes, genes with specific high expression in pluripotent stem cells were selected using KEGG and GO pathway analysis databases. The analysis results are shown in Table 4 and Figure 1.

**Table 4**

| **A. Biological Process** | | | |
|---|---|---|---|
| **Item** | **Gene Count** | **Item** | **Gene Count** |
| Cellular processes | 6057 | Multiple biological interaction processes | 1036 |
| Single biological process | 5461 | Biological adhesion | 947 |
| Biological regulation | 4819 | Movement | 664 |
| Regulation of biological processes | 4599 | Reproduction | 623 |
| Metabolic processes | 4516 | Reproductive processes | 621 |
| Response to stimulus | 3508 | Growth | 524 |
| Multicellular organism processes | 3248 | Behavior | 369 |
| Developmental processes | 2896 | Rhythmic processes | 176 |
| Signaling | 2817 | Presynaptic processes involved in chemical synaptic transmission | 90 |
| Cellular component structure or biogenesis | 2770 | Detoxification | 54 |
| Localization | 2696 | Apoptosis | 39 |
| Positive regulation of biological processes | 2489 | Cell aggregation | 17 |
| Negative regulation of biological processes | 2276 | Biological phase | 7 |
| Immune system processes | 1126 | | |

| **B. Cellular components** | | | |
|---|---|---|---|
| **Item** | **Gene Count** | **Item** | **Gene Count** |
| Cells | 6444 | Cell junctions | 752 |
| Cellular components | 6435 | Synapses | 534 |
| Organelles | 5342 | Synapse components | 453 |
| Cell membrane | 3805 | Supramolecular complexes | 417 |
| Organelle components | 3304 | Virions | 56 |
| Cell membrane parts | 2901 | Virion components | 56 |
| Extracellular region | 2000 | Nucleocapsids | 31 |
| Macromolecular complexes | 1912 | Other organisms | 27 |
| Extracellular region components | 1774 | Other organism components | 27 |
| Cell membrane-bounded compartments | 1751 | | |

| **C. Molecular functions** | | | |
|---|---|---|---|
| **Item** | **Gene Count** | **Item** | **Gene Count** |
| Binding | 5878 | Molecular transducer activity | 587 |
| Catalytic activity | 2529 | Structural molecule activity | 349 |
| Nucleic acid transcription factor | 733 | Transcription factor binding | 285 |
| Transporter activity | 625 | Antioxidant activity | 36 |
| Signal transducer activity | 615 | Translation regulator activity | 31 |
| Molecular function regulator | 607 | Metal chaperone protein | 2 |

### Example 2: Analysis of Differentially Expressed Genes and Screening of Candidate Genes for Pluripotent Stem Cell Residue

Using the MiniBEST Universal RNA Extraction Kit (Catalog No. 9767) from TaKaRa, total RNA was extracted from induced pluripotent stem cells, induced dopamine precursor cells (DPCs), induced dopamine neuron cells, and induced photoreceptor cells according to the manufacturer's instructions. RNA purity was assessed using a Nanodrop (OD260/280 ratio), and qualified mRNA samples were used for differential expression gene analysis. Based on the experimental data, the genes ESRG, TDGF1, ESRP1, ZSCAN10, PRDM14, TRIML2, AP1M2, NR5A2, and SCNN1A, with log2(FC) < -10 in DPC cells, were selected as candidate genes for detecting pluripotent stem cell residues in DPCs. These candidate genes were compared against the commonly used markers Oct4, Nanog, and Lin28. The analysis results are shown in Table 5.

**Table 5. Candidate Gene Indicators for Residual Pluripotent Stem Cells in DPC**

| **Gene** | **Average value (Base mean)** | **log2(FC)** | **Adjusted P-value (P-adj)** |
|---|---|---|---|
| ESRG | 15346.70541 | -13.8897147 | 2.61E-143 |
| TDGF1 | 6458.616019 | -13.5901229 | 1.07E-78 |
| ESRP1 | 1925.184993 | -12.0193387 | 3.91E-56 |
| ZSCAN10 | 2618.95944 | -11.7312392 | 9.09E-81 |
| PRDM14 | 1049.299648 | -10.2299186 | 3.00E-68 |
| TRIML2 | 900.4871859 | -11.0424029 | 1.43E-44 |
| AP1M2 | 995.9147347 | -10.159743 | 3.60E-67 |
| NR5A2 | 654.7260957 | -10.6336799 | 3.06E-40 |
| SCNN1A | 1125.878078 | -11.3288057 | 8.94E-48 |
| Oct4 | 29728.71945 | -14.2719184 | 4.79E-216 |
| Nanog | 1954.349583 | -12.0370008 | 2.46E-56 |
| Lin28 | 20089.01 | -5.17795 | 0 |

### Example 3: qPCR Detection of Candidate Gene Expression Levels in Pluripotent Stem Cells and Differentiated Derivatives

Total RNA was extracted from both pluripotent stem cells and DPC cells, then reverse-transcribed into cDNA. qPCR was used to detect the expression levels of candidate genes (ESRG, TDGF1, ESRP1, ZSCAN10, PRDM14, TRIML2, AP1M2, NR5A2, SCNN1A) related to pluripotent stem cell residuals in DPC cells. The data were compared with the commonly used markers Oct4, Nanog, and Lin28. The upstream and downstream primer sequences for the candidate genes are shown in Table 6, and the expression Ct values of the candidate genes are shown in Table 7. qPCR results indicated that these candidate genes were highly expressed in pluripotent stem cells compared to DPC cells. However, there were significant differences in baseline expression levels among the genes in DPC cells. TDGF1 and TRIML2 had very low baseline expression (Ct values 38-N/A), whereas Oct4, Nanog, and Lin28 had relatively high baseline expression (Ct values 21-25), with minimal differences compared to iPSC cells.

The nucleotide sequence length of the TDGF1 gene is 1956 bp, as shown in SEQ ID NO: 1; the sequence length of the TRIML2 gene is 1998 bp, as shown in SEQ ID NO:2.

**Table 5. Candidate Gene Indicators for Residual Pluripotent Stem Cells in DPC**

| **Gene** | **SEQ ID** | **Primer Type** | **Primer Sequence** |
|---|---|---|---|
| TDGF1 | 3 | Forward Primer | GGCTGCTGCTACAATGTCCTAACT |
| | 4 | Reverse Primer | GGCACAATGGAGATATGGCTGACT |
| TRIML2 | 5 | Forward Primer | CACGGCTGTTCTGTGATGTTGAC |
| | 6 | Reverse Primer | TCCTCTTCCTGAATCATCGCCATT |
| ACTIN | 7 | Forward Primer | TCGTGCGTGACATTAAGGAG |
| | 8 | Reverse Primer | TTGCCAATGGTGATGACCTG |
| ESRG | 9 | Forward Primer | GCCTGAACTGTAGCAGCCAGAC |
| | 10 | Reverse Primer | CAGCCAGTAAGCCAAGAAGGAGTC |
| ESRP1 | 11 | Forward Primer | GCTGGTGGTACTTCCAATGAGGTA |
| | 12 | Reverse Primer | CCATCTGGGTAGGTGACAAAGAGG |
| ZSCAN10 | 13 | Forward Primer | TCGCACCTGAGCAAGCACCT |
| | 14 | Reverse Primer | GGAGCACAGGACATCGGTCAGT |
| PRDM14 | 15 | Forward Primer | GGTTCACAGCCTCCAGCATACTC |
| | 16 | Reverse Primer | TGAGCAGCCATCATCCTCCTTGT |
| AP1M2 | 17 | Forward Primer | TCTGTCAACCTGCTGGTCAATGC |
| | 18 | Reverse Primer | TGAAGGAGATGGTGCGGTCGTT |
| NR5A2 | 19 | Forward Primer | GCAGTCTCAGTCACCCGTGTTATC |
| | 20 | Reverse Primer | GTTGACCACACCATTCCTCTCCAT |
| SCNN1A | 21 | Forward Primer | GGTGGACTGGAAGGACTGGAAGA |
| | 22 | Reverse Primer | TTGAAGCGGCAGGCGAAGATG |
| Oct4 | 23 | Forward Primer | ATCTTCAGGAGATATGCAAAGCAGA |
| | 24 | Reverse Primer | TGATCTGCTGCAGTGTGGGT |
| Nanog | 25 | Forward Primer | ACCTCAGCTACAAACAGGTGAA |
| | 26 | Reverse Primer | AAAGGCTGGGGTAGGTAGGT |
| Lin28 | 27 | Forward Primer | TTCTGCCATAGTGTCCTCTTGA |
| | 28 | Reverse Primer | AGCCTCTCCTAGCCTCTGT |

**Table 7. Ct Values for the Expression of Candidate Genes for Residual Pluripotent Stem Cells**

| **Gene** | **Ct (Pluripotent stem cells**) | **Ct (DPC)** | **Gene** | **Ct** (**Pluripoten t stem cells**) | **Ct (DPC)** |
|---|---|---|---|---|---|
| ACTIN | 12.863 | 15.062 | PRDM14 | 21.368 | 34.368 |
| | 12.484 | 15.036 | | 21.337 | 35.218 |
| | 12.467 | 15.044 | | 21.333 | 34.889 |
| ESRG | 13.465 | 28.280 | TRIML2 | 21.461 | N/A |
| | 13.53 | 28.664 | | 21.405 | N/A |
| | 13.436 | 28.603 | | 21.435 | N/A |
| TDGF1 | 17.476 | N/A | AP1M2 | 18.725 | 36.114 |
| | 17.375 | 38.516 | | 18.800 | 38.136 |
| | 17.384 | N/A | | 18.709 | 35.171 |
| ESRP1 | 19.180 | 32.510 | NR5A2 | 25.352 | 35.085 |
| | 19.041 | 32.024 | | 25.348 | 34.792 |
| | 19.036 | 32.725 | | 25.406 | 34.555 |
| ZSCAN10 | 22.986 | 30.209 | SCNN1A | 20.151 | 29.975 |
| | 23.833 | 31.214 | | 20.203 | 30.195 |
| | 23.276 | 30.714 | | 20.165 | 29.818 |
| Oct4 | 16.909 | 21.966 | Nanog | 22.294 | 25.117 |
| | 16.777 | 21.735 | | 22.314 | 25.161 |
| | 16.913 | 21.799 | | 22.391 | 25.116 |
| Lin28 | 21.573 | 21.832 | | | |
| | 21.682 | 21.838 | | | |
| | 21.497 | 21.765 | | | |

Based on the RNA-seq data from Example 1 (Table 5) and the qPCR results from Example 2 (Table 7), genes that have low expression levels in dopamine precursor cells but high baseline levels in pluripotent stem cells were selected as marker genes for detecting pluripotent stem cell residues in dopamine precursor cells. These genes are TDGF1 and TRIML2.

### Example 4: Validation of Pluripotent Stem Cell Residue Detection in Dopamine Precursor Cells (DPC) from Standard Samples

Standard Sample Preparation: Mix 0, 10², 10³, and 10⁴ pluripotent stem cells with 10⁶ DPC cells, with three biological replicates for each sample. Centrifuge at 2000 rpm for 5 minutes, discard the supernatant, and collect the pellet. Extract total RNA, then reverse transcribe it to cDNA. Finally, perform quantitative PCR to obtain the Ct values of TDGF1 and TRIML2 gene expression for pluripotent stem cell ratios of 0, 0.01%, 0.1%, and 1% in DPC.

Quantitative PCR Results: The Ct values for TDGF1 gene expression in DPC with pluripotent stem cell ratios of 0, 0.01%, 0.1%, and 1% are 34.039, 31.369, 27.963, and 23.979, respectively. The Ct values for TRIML2 gene expression in DPC with pluripotent stem cell ratios of 0, 0.01%, 0.1%, and 1% are 33.905, 34.194, 30.667, and 27.478, respectively.

**Table 8: Ct Values for TDGF1 and TRIML2 Gene Expression in Standard Samples**

| **Pluripotent Stem Cell Ratio** | **Ct (*Actin***) | **Average Value** | **Ct (*TDGF1*)** | **Average Value** | **Ct (*TRIML2***) | **Averag e Value** |
|---|---|---|---|---|---|---|
| 0 | 14.483/14.4 37/14.416 | 14.445 | 34.408/33.73 6/33.973 | 34.039 | 33.382/34.011/ 34.321 | 33.905 |
| 0.01% | 14.253/14.1 38/14.292 | 14.228 | 31.437/31.35 2/31.319 | 31.369 | 34.276/34.601 /33.705 | 34.194 |
| 0.1% | 14.402/14.3 81/14.196 | 14.326 | 28.011/ 27.8/28.079 | 27.963 | 30.716/30.37/ 30.915 | 30.667 |
| 1% | 14.309/14.3 97/14.373 | 14.360 | 23.98/23.959/ 23.998 | 23.979 | 27.519/27.663 / 27.252 | 27.478 |

### Example 5: Detection of Residual Pluripotent Stem Cells in Dopaminergic Progenitor Cells (DPC) Derived from Pluripotent Stem Cells

In this example, inducible directed differentiation DPCs are used as samples to detect residual pluripotent stem cells. The samples to be tested include 1 × 10^6 DPC-1, 1 × 10^6 DPC-2, and 1 × 10^6 DPC-3. cDNA is prepared from samples DPC-1, DPC-2, and DPC-3 according to the methods outlined for total mRNA extraction and reverse transcription. The cDNA extracted from the three independent batches of DPC-1, DPC-2, and DPC-3 is subjected to qPCR analysis, and the Ct values for TDGF1 and TRIML2 gene expression in the samples are obtained. The results are shown in Table 9.

**Table 9: Ct Values for TDGF1 and TRIML2 Gene Expression in the Samples**

| **Testing Samples:** | **Ct (*Actin*)** | **Average Value** | **Ct (*TDGF1*)** | **Average Value** | **Ct** (***TRIML2**)* | **Average Value** |
|---|---|---|---|---|---|---|
| DPC-1 | 14.474/14.67 4/14.678 | 14.609 | 35.856/34.36 3/34.147 | 34.789 | 37.397/NA/3 9.058 | 38.228 |
| DPC-2 | 15.112/14.97 /14.955 | 15.012 | 34.846/34.88 5/35.371 | 35.034 | 37.735/38.20 5/36.95 | 37.630 |
| DPC-3 | 15.03/15.059 /15.064 | 15.051 | 37.083/ 36.07/35.942 | 36.365 | 37.575/38.90 9/NA | 38.242 |

Based on the results from the quantitative PCR, the Ct values for the TDGF1 gene in DPC-1, DPC-2, and DPC-3 are 34.789, 35.034, and 36.365, respectively. The Ct values for the TRIML2 gene in the same samples are 38.228, 37.630, and 38.242, respectively. The Ct values for actin in both the standard samples (Table 8) and the test samples (Table 9) are nearly identical. Since TDGF1 and TRIML2 are highly expressed in pluripotent stem cells, the Ct values of TDGF1 and TRIML2 in the standard samples are compared with those in the test samples. The standard curve formulas are as follows:
1 × 10^6 DPC + 10^2 pluripotent stem cells is equivalent to 0.01%;
1 × 10^6 DPC + 10^3 pluripotent stem cells is equivalent to 0.1%;
1 × 10^6 DPC + 10^4 pluripotent stem cells is equivalent to 1%.

From these formulas, it can be calculated that the residual pluripotent stem cells in different batches of DPC cells (DPC-1, DPC-2, DPC-3) are close to or less than 0.01%, i.e., one in ten thousand.

Example 6: Comparative Analysis of TDGF1 and TRIML2 Expression Levels in Differentiated Cells from Other Pluripotent Stem Cell Sources

In this example, retinal progenitor cells (RPC), hepatic progenitor cells (HPC), and cardiomyocyte progenitor cells (CPC) derived from induced pluripotent stem cells are used as samples. The expression levels of TDGF1 and TRIML2 are detected and compared with those in DPC cells to determine if these genes are suitable for detecting residual pluripotent stem cells in these other cell types. The samples tested are 1 × 10^6 iPSC, 1 × 10^6 RPC, 1 × 10^6 HPC, 1 × 10^6 CPC, and 1 × 10^6 DPC-1. Total RNA is extracted and reverse transcribed into cDNA, and qPCR is performed on the cDNA to obtain the Ct values for TDGF1 and TRIML2 in the samples. The results are shown in Table 10.

**Table 10: Ct Values for TDGF1 and TRIML2 Gene Expression in Samples**

| **Testing Samples:** | **Ct (*Actin*)** | **Average Value** | **Ct (*TDGF1*)** | **Average Value** | **Ct (*TRIML2*)** | **Average Value** |
|---|---|---|---|---|---|---|
| iPSC | 14.45/14.51 | 14.48 | 17.476/17.375 /17.384 | 17.412 | 21.461/21.4 05/21.435 | 21.433 |
| DPC-1 | 14.474/14.67 4/14.678 | 14.609 | 35.856/34.363 /34.147 | 34.789 | 37.397/NA/ 39.058 | 38.228 |
| RPC | 15.09/15.18 | 15.14 | 33.15/33.15 | 33.15 | 38.96/39.02 | 38.99 |
| HPC | 15.20/15.14 | 15.17 | 27.69/ 28.43 | 28.06 | 29.35/ 29.39 | 29.37 |
| CPC | 16.89/17.05 | 16.97 | 29.87/ 29.81 | 29.84 | 29.69/ 29.72 | 29.70 |

From the quantitative PCR results, it can be seen that, compared to iPSC cells, the Ct values for the TDGF1 gene in DPC-1, RPC, HPC, and CPC cells are relatively close and are significantly lower than the TDGF1 gene expression level in iPSC cells (Ct value of 17.412). Similarly, the Ct values for the TRIML2 gene in the four different differentiated cells are also relatively close and significantly lower than the TRIML2 gene expression level in iPSC cells (Ct value of 21.433). Therefore, using TDGF1 and TRIML2 as marker genes for detecting pluripotent stem cell residue is also applicable in these differentiated cells.

The invention is not limited to the specific details, representative embodiments, and examples described herein. Those skilled in the art will recognize that various modifications and other advantages can be achieved without departing from the spirit and scope of the claims and the general concept defined.

## Claims

1. The use of marker genes in detecting residual pluripotent stem cells, wherein the marker genes are used to detect residual pluripotent stem cells in pluripotent stem cell-derived cells, wherein the marker genes include at least one of TDGF1 and TRIML2.

2. The use of claim 1, wherein the pluripotent stem cell-derived cells include neural stem cells, retinal ganglion cells, retinal pigment epithelial cells, liver cells, natural killer cells, and cardiomyocytes.

3. A method for detecting residual pluripotent stem cells, wherein the detection method includes using at least one marker gene selected from TDGF1 and TRIML2 to detect the amount of residual pluripotent stem cells.

4. The method of claim 3, wherein the method involves quantitatively detecting the marker gene in the sample and comparing it with a standard of pluripotent stem cell-derived cells containing known quantities of pluripotent stem cells to obtain the amount of residual pluripotent stem cells.

5. The method of claim 4, wherein it includes the following steps:
(1) Adding varying gradients of pluripotent stem cells to pluripotent stem cell-derived cells to create standards;
(2) Designing primers based on the sequences of the marker genes and performing quantitative PCR reactions with the primers and the standards to create a standard curve for residual pluripotent stem cells in the pluripotent stem cell-derived cells;
(3) Performing quantitative PCR on the pluripotent stem cell-derived cells to detect the expression levels of the marker genes in the sample, and determining the residual pluripotent stem cell proportion based on the comparison of Ct values.

6. The method of claim 5, wherein the nucleotide sequences of the primers for the marker gene TDGF1 are SEQ ID NO: 3 and/or SEQ ID NO: 4.

7. The method of claim 5, wherein the nucleotide sequences of the primers for the marker gene TRIML2 are SEQ ID NO: 5 and/or SEQ ID NO: 6.

8. The method of claim 5, wherein in step (2), the qPCR detection program is: pre-denaturation at 95°C for 120 seconds; PCR reaction, 40 cycles of 95°C for 10 seconds, 58°C for 20 seconds; followed by 94°C for 30 seconds.

9. A reagent kit for detecting residual pluripotent stem cells, wherein it includes primers designed based on the nucleotide sequences SEQ ID NO: 1 or SEQ ID NO: 2.

10. The reagent kit of claim 9, wherein the primers include at least one of the nucleotide sequences SEQ ID NO: 3-4 or SEQ ID NO: 5-6.
